Europäisches Patentamt

European Patent Office

Office européen des brevets

(11) Veröffentlichungsnummer: **0 224 604**
**A1**

(12) # EUROPÄISCHE PATENTANMELDUNG

(21) Anmeldenummer: 85115241.3

(51) Int. Cl.⁴: **A61B 5/14**

(22) Anmeldetag: 30.11.85

(43) Veröffentlichungstag der Anmeldung:
**10.06.87 Patentblatt 87/24**

(84) Benannte Vertragsstaaten:
**AT BE CH DE FR GB IT LI LU NL SE**

(71) Anmelder: **INTERMEDICAT GMBH**
**Gerliswilstrasse 74**
**CH-6020 Emmenbrücke(CH)**

(72) Erfinder: **Schwöbel, Eckhardt, Dipl.-Ing.**
**Reckenbühlstrasse 17**
**CH-6005 Luzern(CH)**
Erfinder: **Bäumle, Hubert**
**Wanne**
**CH-6182 Escholzmatt(CH)**

(74) Vertreter: **Maspoli, Renato A.,**
**Dipl.-Chem.Ing.ETH**
**Patentanwälte R.A. Maspoli und Partner**
**Promenadengasse 18**
**CH-8001 Zürich(CH)**

(54) **Blutentnahme-Gerät mit Sicherheitsventil und Zylinderbehälter.**

(57) Das neue Sicherheits-Blutentnahmegerät umfasst als Hauptbestandteile einen Spritzenkörper - (SK) mit Kanülenhalter, einen in den Spritzenkörper einzudrückenden Auffangbehälter (AB) sowie ein durchgehendes Rohr (R).

Der Auffangbehälter liegt in Form eines Hohlzylinders (11) mit vorn angebrachtem, fixiertem Abdichtungspfropfen (12) aus elastischem Material und mit innen angebrachtem, gegenüber der Kolbeninnenwand beweglichem Abdichtungskolben (13) vor.

Der Spritzenkörper weist an seinem hinteren Ende Grifflappen (21) und an der Stirnfläche (22) einen Kanülenhalter (23) auf.

Das durchgehende Rohr ist mittels eines Abschlussstückes (31) in einem Verschlussblock (32) in Richtung Geräte-Längsachse beweglich gelagert, wobei der Verschlussblock (32) dicht an der vorderen Abschliessfläche (22) befestigt ist und eine flüssigkeitsführende Verbindung zwischen Kanüleninnenraum und innerem Hohlraum (33) des Verschlussblockes (32) schafft, und wobei das am durchgehenden Rohr dicht fixierte Abschlussstück (31) bei Druck auf das Rohr in Richtung Gerätespitze eine flüssigkeitsführende Verbindung zwischen dem Rohr-Innenraum und dem inneren Hohlraum (33) schafft, und bei Zug auf das durchgehende Rohr diese Verbindung flüssigkeitsdicht abschliesst.

Verwendet wird das neue Gerät, vorteilhafterweise als Set, für die Blutentnahme bei Menschen.

Fig. 1

## BLUTENTNAHME-GERAET MIT SICHERHEITSVENTIL UND ZYLINDERBEHAELTER

Die im Folgenden beschreibene Erfindung betrifft ein gesichertes Blutentnahmegerät mit Behälterkolben in Form einer einhändig zu betätigenden und zu kontrollierenden Spritze, in die, zwecks Betätigung, jeweils ein einmalig zu verwendender abgedichteter Zylinder, der nach der Entnahme des Blutes zugleich als dessen Behälter dient, eingedrückt wird.

Blutentnahme-Geräte sind seit langem bekannt; da jedoch die Blutprobenahme als Teil von Diagnostik-Verfahren gemäss Aussagen von Fachleuten sicher zunehmen wird, ist der Bedarf nach gesicherten, einfach zu betätigenden und hygienisch einwandfrei zu produzierenden und zu lagernden Blutentnahmegeräten bestimmt gegeben.

Der Stand der Technik gattungsgemässer Geräte kann in zwei Gruppen aufgeteilt werden: Blutentnahme-Spritzen, bei denen die zur Blutentnahme aus Venen notwendige Druckanpassung, bzw. der Unterdruck durch manuelles Verschieben eines Kolbens in einem Zylinder erzeugt wird und Geräte, bei denen der genannte Unterdruck unter Verwendung von evakuierten Behältern oder eines entleerten Gummiballons geschaffen wird.

Die erste Gruppe der Apparate dieser Art werden gut durch die Erfindung gemäss dem EP-Gesuch, Ver. Nr. 0 107 578, dargestellt.

Mittels eines von Hand nach hinten bewegten Kolbens wird im Spritzenkörper ein Unterdruck erzeugt, welcher Unterdruck bewirkt, dass Blut durch die vorgeschaltete Kanüle in das zwischen der Kanüle und dem Spritzenkörper angebrachte Behälterrohr gesaugt wird. Eine Sicherung hinsichtlich Wiederausstossen des Blutes ist nicht vorgesehen und die Vorrichtung wird anscheinend für jede neue Blutentnahme auseinandergenommen.

Auch die Erfindung gemäss EP-Anmeldung, Ver Nr. 0 150 127, gehört im Grunde genommen zu dieser Gerätegruppe: Durch das Eindrücken des Hohlkolbens in den Spritzenkörper wird im Hohlzylinder selbst, durch die Verschiebung eines darin angebrachten Gleitkolbens, ein Unterdruck erzeugt, welcher dazu dient, ein praktisch an der Kanüle angebrachtes Behältergefäss mit Flüssigkeit zu füllen.

Die Nachteile derartiger Geräte sind einmal die ungenügende Sicherung gegen das Ausstossen der Flüssigkeit während oder nach der Entnahme und, bei zweihändig· betätigten Spritzen für die Blutentnahme, die Unmöglichkeit, eine Hand für die Ruhigstellung z.B. des Armes des Patienten freizubehalten. Beim Gerät gemäss EP-Gesuch, Ver. Nr. 0 150 127, ist zudem noch auf die Lage des Behälters hinzuweisen: Der Spiegel der darin aufgenommenen Flüssigkeit ist jedenfalls tiefer als der hydrostatische Punkt der Unterdruckleitung. Eine praktische Blutentnahme mit dieser Spritze ist nicht leicht ersehbar.

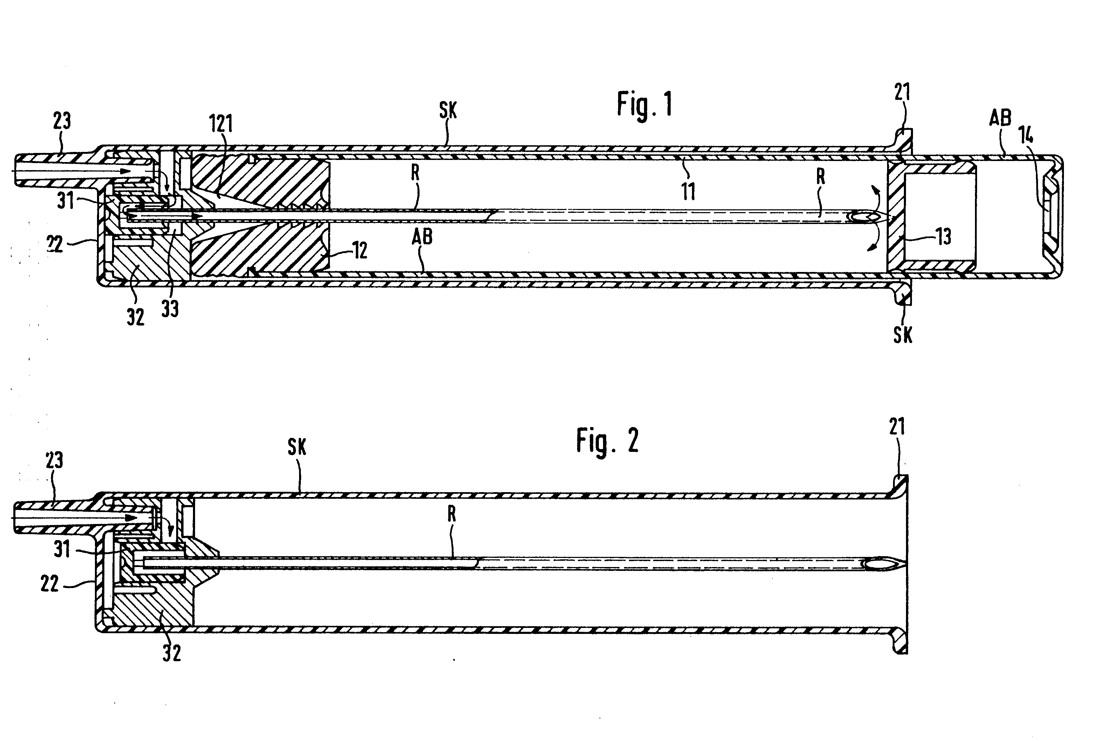

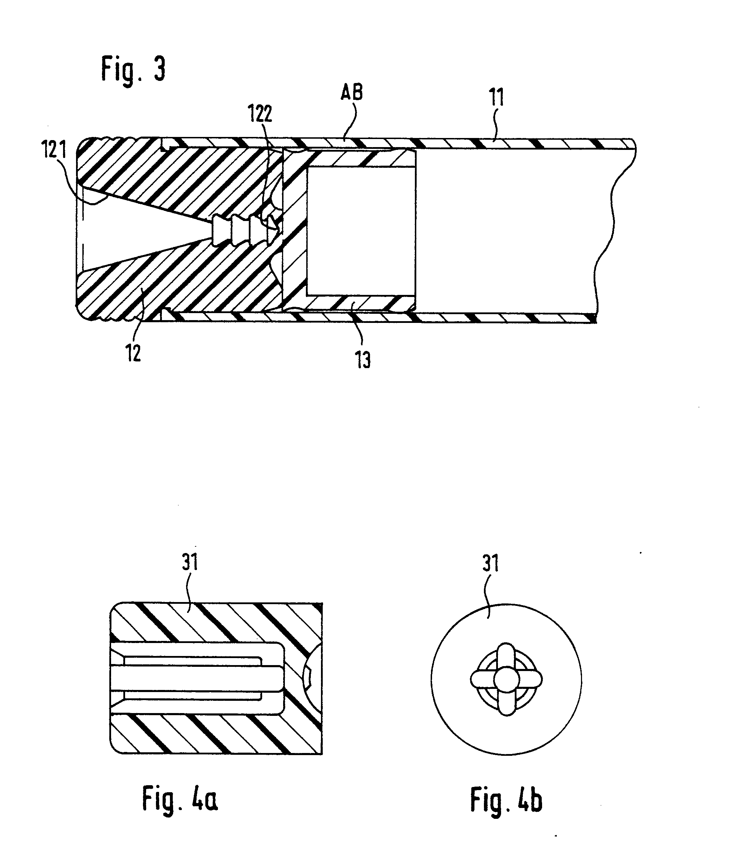

Die zweite Gruppe derartiger Geräte gemäss dem Stand der Technik wird repräsentiert u.a. durch die Erfindung gemäss EP-Gesuch, Ver. Nr. 0 077 441, speziell in den Figuren 1 und 3: Ein Halter weist beidseitig Nadeln auf, wovon die eine als eigentliche Kanüle, die andere zum Anstechen eines hinten anzubringenden, vorevakuierten Auffangbehälters benutzt wird. Die EP-Anmeldung, Ver. Nr. 0 045 863, lehrt, anstelle eines evakuierten Behälters, einen Gummiballon, der jeweils entleert wird und bei der Blutentnahme zur Erzeugung des Unterdruckes im dazwischen geschalteten, aufgesteckten Behälter dient.

Die Nachteile derartiger Systeme sind die plötzliche und praktisch unkontrollierte Einwirkung von relativ hohem Unterdruck, was zum Kollabieren der Venen führen kann. Zudem ist die Lagerzeit von vorevakuierten Behältern begrenzt und diese Dauer erst noch mit einer gewissen Unsicherheit behaftet. Keine der Apparaturen weist schliesslich eine Rück-Austrittssicherung auf.

Das vorliegend beschriebene, erfindungsgemässe Blutentnahmegerät umgeht, durch neue konstruktive und anordnungsmässige Eigenheiten, die oben genannten Schwierigkeiten und Nachteile. Vor allem kommt die Blutentnahmevorrichtung gemäss der Erfindung dem Ideal hinsichtlich Kontamination, d.h. der einmaligen Verwendung einer Vorrichtung für eine Blutentnahme, praktisch sehr nahe. Die mehrmalig eingesetzten, mit dem Blut in Verbindung kommenden Bestandteile können alle aus Spezialstahl, bzw. aus speziell oberflächenbehandeltem Stahl sein.

Die in dieser Beschreibung im Zusammenhang mit Blutentnahmegeräten verwendeten Bezeichnungen "vorn" und "hinten" beziehen sich auf die übliche Verwendungsart solcher Geräte: "vorn" deutet auf die Richtung der Kanüle bzw. des Einstiches hin, "hinten" auf das entgegengesetzte Ende des Apparates in Richtung dessen Längsachse.

Diese Erfindung betrifft speziell ein gesichertes Blutentnahmegerät, welches als Hauptbestandteil einen Spritzenkörper SK mit Kanülenhalter, einen in den Spritzenkörper einzudrückenden Auffangbehälter AB sowie ein durchgehendes Rohr R umfasst. Das Gerät ist dadurch gekennzeichnet, dass der Auffangbehälter in Form eines Hohlzylinders 11 mit vorn angebrachter, fixierter Abdichtung 12 aus elastischem Material, mit innen angebrachtem, gegenüber der Zylinderinnenwand beweglichem Abdichtungskolben 13 und mit hinten vorgesehener

Oeffnung 14, welche beim Eindrücken des Hohlzylinders in den Spritzenkörper unter Verschiebung des Abdichtungskolbens das Austreten der Luft ermöglicht, vorliegt, dass der Spritzenkörper an seinem hinteren Ende Grifflappen 21, die bei der Verwendung des Gerätes z.B. durch Zeig-und Mittelfinger gehalten werden und an der vorderen Stirnfläche 22 einen Kanülenhalter 23 aufweist sowie dass der Spritzenkörper am hintern Ende offen ausgebildet ist und dass das durchgehende Rohr mittels eines Abschlussstückes 31 in einem Verschlussblock 32 in Richtung Geräte-Längsachse beweglich gelagert ist, wobei der Verschlussblock 32 dicht an der Stirnfläche 22 befestigt ist und eine flüssigkeitsführende Verbindung zwischen Kanüleninnenraum und innerem Hohlraum 33 des Verschlussblockes 32 schafft, und wobei das am durchgehenden Rohr dicht fixierte Abschlussstück 31 bei Druck auf das Rohr in Richtung Gerätespitze eine flüssigkeitsführende Verbindung zwischen dem Rohr-Innenraum und dem inneren Hohlraum 33 schafft und bei Zug auf das durchgehende Rohr diese Verbindung flüssigkeitsdicht abschliesst.

Das neue Blutentnahmegerät weist vorteilhafterweise einen Auffangbehälter aus spritzgeformtem Polyolefin-Material auf sowie eine Abdichtung 12 in Form eines Pfropfens aus keimfrei zu machendem Synthese-oder Naturkautschuk. Diese Abdichtung zeigt eine der Lage des durchgehenden Rohres entsprechende Vertiefung 121 und Dünnstelle 122 zur Führung des Rohres und zur Bestimmung der Durchstichstelle, wobei die genannte Abdichtung über das vordere Ende des Auffangbehälters hinausragt und zugleich zur nichtdichtenden Führung dieses Behälters im Spritzenkörper dient. Ebenso liegt in diesem Auffangbehälter ein innerer beweglichen, ebenfalls aus Polyolefinen spritzgeformter Abdichtungkolben 13 vor, der in seinem Aussendurchmesser und in seiner Anlagefläche derart an den Auffangbehälter angepasst ist, dass er bei den auftretenden Druck unterschieden für die üblicherweise aufzunehmenden Flüssigkeiten dicht ist. Der Auffangbehälter ist zudem so ausgebildet, dass er -leer oder gefüllt - auf die hintere Abschlussfläche gestellt werden kann; er muss also nicht gelegt werden.

Das gleiche Blutentnahmegerät weist bevorzugterweise einen Spritzenkörper aus Polyolefin-Material auf, der druckgeformt ist, und er enthält einen material-und formmässig integrierten Kanülenhalter 23. Die Kanüle kann übrigens eine Stahlkanüle oder eine Stahlspitze mit einer elastischen Zuleitung zum Kanülenhalter sein.

Weiter ist das vorliegend beschriebene Blutentnahmegerät ein solches, das ein durchgehendes Rohr aus Spezialstahl enthält, welches Rohr vorn ein Abschlussstück 31 vornehmlich aus spritzgeformten Acryl-Kunststoffen aufweist, das die flüssigkeitsführende Verbindung vom Rohr-Innenraum zum inneren Hohlraum 32 des Verschlussblockes herstellt bzw. unterbricht und dicht und nichtbeweglich am Rohrende aufgezogen ist.

Dieses Stahlröhrchen ist mittels des Abschlussstückes 31 im Verschlussblock 32 axial beweglich gelagert, wobei der Verschlussblock vorn, z.B. innen an der Stirnfläche 22 des Spritzenkörpers befestigt, z.B. angeklebt, ist und die flüssigkeitsführende Verbindung zwischen dem Kanüleninnenraum und -bei Druck auf das durchgehende Rohr -seinem inneren Hohlraum 33 - schafft.

Das genannte Abschlussstück 31 -und somit das Rohr -sind flüssigkeitsdicht im genannten Hohlraum 33 in Achsenrichtung beweglich gelagert. Das durchgehende Rohr ist hinten schräg angeschnitten, um so bei der Verwendung des Gerätes das Einstechen des genannten Rohres in die Dünnstelle 122 der Abdichtung 12 zu erleichtern.

Das erfindungsgemässe Blutentnahmegerät liegt vorteilhafterweise in Form eines Sets vor. Darin sind sowohl der Spritzenkörper mit dem darin befestigten durchgehenden Rohr wie auch der bzw. die Auffangbehälter verpackt. Speziell kann im genannten Set der Spritzenkörper mit durchgehendem Rohr in einem separaten Abteil gelagert sein, welches Abteil vom übrigen Set-Behälter leicht - z.B. durch Abreissen -abtrennbar ist, währenddem der oder die Auffangbehälter gemeinsam in einem zweiten Abteil gelagert ist (sind), welches zweite Abteil zudem, nach Abtrennen des separaten Abteils zusammen mit dem gebrauchten Spritzenkörper, beschrift-und versendbar ist.

Verwendet wird das gesicherte Blutentnahmegerät gemäss der Erfindung zur Blutentnahme bei Menschen. Diese Verwendung kann z.B. die folgenden Schritte umfassen:
-Einführen der vorerst am Blutentnahmegerät starr oder beweglich angebrachten Kanüle in die Vene,
-Eindrücken eines Auffangbehälters in den Spritzenkörper, wobei das durchgehende Rohr die vordere Abdichtung 12 aus elastischem Material durchsticht und den inneren; beweglichen Abdichtungskolben 13 im Auffangbehälter nach hinten - schiebt und so darin einen Unterdruck erzeugt, welcher Unterdruck, da mit dem Druck auf das Rohr in Richtung Gerätespritze das Abschlussstück 31 nach vorn geschoben wird und so die Verbindung Kanülen-Innenraum 33 -Rohr geschaffen wird, das Aufsaugen von Blut in den Auffangbehälter bewirkt und
-Zurückziehen des Auffangbehälters bei Erreichen der gewünschten Blutmenge im Auffangbehälter, wodurch sowohl Blutzufuhr wie auch -austritt gestoppt werden und wodurch, beim vollständigen Zurückziehen des Auffangbehälters, ein hermetisch verschlossener Behälter mit dem entnommenen Blut erhalten wird.

Bei der genannten Verwendung kann die eigentliche Blutentnahme bei gleicher eingestochener Kanüle mit gleichem Spritzenkörper und durchgehendem Rohr nacheinander unter Verwendung von verschiedenen Auffangbehältern, beispielsweise unter Verwendung des oben beschriebenen Sets, mehrmals ausgeführt werden.

Aus den obigen allgemeinen und speziellen Darstellungen dieser Erfindung folgern jetzt auch klar die Unterscheidungsmerkmale gegenüber dem Stand der Technik.

Speziell hinsichtlich der EP-Anmeldung, Ver. Nr. 0 150 127, in der ein einhändig zu betätigendes Gerät -in Form einer Spritze -zur Erzeugung von Unterdruck beschrieben und das u.a. auch für die Entnahme von Blut und zur Aufnahme desselben in einem separat angeschlossenen Behälter geeignet ist, ist folgendes anzumerken: Die aufgesaugte Flüssigkeit sollte darin nie in den eigentlichen Gerätekörper eintreten, v.a. nicht in den zur Erzeugung der Unterdruckes benötigten, in den Spritzenkörper eingedrückten Hohlkolben. Dieser Kolben gehört zum Gerät, er wird bei jeder Betätigung desselben eingedrückt. Nach der Flüssigkeitsentnahme wird -ohne jegliche Sicherung -der separate Behälter weggenommen und der Kolben wieder aus dem Spritzenkörper zurückgezogen, wodurch sich das den Unterdruck produzierende variable Volumen im genannten Kolben wieder auf praktisch Null reduziert.

Ebenso ist zu dieser Veröffentlichung anzumerken, dass keine Aussagen gemacht werden hinsichtlich der in Ausführung der Erfindung jeweils praktisch zu verwendenden Materialien bzw. Werkstoffe und der Herstellungs-bzw. Bearbeitungsverfahren derselben. Dies ist jedoch in derartigen Geräten von ausschlaggebender Bedeutung.

Demgegenüber weist die erfindungsgemässe Blutentnahme-Vorrichtung einen als Blut-Aufnahme und -Behälter ausgebildeten und demzufolge nur einmal zu verwendenden Hohlzylinder auf. Ebenso ist das axiale Durchtrittsröhrchen bei der Spitze der Vorrichtung mit einer Abdichtungsvorrichtung versehen, welche Abdichtung beim Eindrücken des Zylinders, d.h. der Unterdruck-Erzeugung automatisch öffnet und beim Zurückziehen des Behälterzylinders flüssigkeitsdicht schliesst.

Das erfindungsgemässe Blutentnahmegerät ist material-und herstellungsmässig derart konzipiert, dass es -bzw. seine Bestandteile -mit den üblichen Massnahmen keimfrei gemacht werden kann.

Im Folgenden werden nun das erfindungsgemässe Sicherheits-Blutentnahmegerät und seine Verwendung anhand der beigelegten Figuren 1 bis 4 ausführlich erläutert.

Die genannten Figuren stellen lediglich eine -wenn auch bevorzugte -Ausführungsform der erfindungsgemässen Vorrichtung dar. Der in den Patentansprüchen charakterisierte Erfindungsgedanke kann durch den Fachmann auch anders realisiert werden.

Es zeigen:

Figur 1 einen zentralen Längsschnitt durch ein erfindungsgemässes Blutentnahmegerät nach Vollendung der Blutentnahme (das Blut ist nicht eingezeichnet); SK ist der Spritzenkörper, AB der Auffangbehälter und R das durchgehende Rohr,

Figur 2 den entsprehenden Schnitt durch den Spritzenkörper SK mit eingesetztem durchgehendem Rohr R ohne eingedrückten Auffangbehälter,

Figur 3 einen zentralen Längsschnitt durch den vorderen Teil des noch nicht eingedrückten Auffangbehälters AB und

Figuren 4a und 4b zentrale Längs-bzw. Querschnitte durch das am durchgehenden Rohr vorn befestigte Abschlussstück 31.

Bei der eigentlichen Blutentnahme sind drei Abschnitte zu unterscheiden. In der folgenden Beschreibung derselben wird angenommen, dass das Blutentnahmegerät in Form des oben schon erwähnten Sets vorliegt, d.h. in einer Verpackung, in der in einem abtrennbaren Abteil der Spritzenkörper SK mit eingesetztem durchgehendem Rohr und evtl. die Kanüle und in einem zweiten Abteil der, bzw. die Auffangbehälter vorliegen; alle Teile keimfrei vorbehandelt und gegebenenfalls noch einzeln verpackt.

Als erstes wird -nach den notwendigen hygienischen und bakteriologischen Vorbereitungen -die Kanüle am Spritzenkörper angebracht und hierauf in die Vene des Patienten eingestochen. Bei fixer Befestigung der Kanüle auf dem Spritzenkörper SK wird dieser selbst zum Einstechen verwendet; bei Benutzung eines Perfusionsbesteckes wird dessen Schlauchverbindung auf den Kanülenhalter 23 aufgesteckt, die Kanüle in die Vene eingeführt und mit den Befestigungsklebern an der Haut fixiert.

Der Spritzenkörper liegt nun grundsätzlich gemäss Figur 2 vor; im Spritzenkörper SK mit Grifflappen 21 (in den Figuren 1 und 2 nur ansatzweise gezeichnet), Stirnfläche 22 und mit integriertem Kanülenhalter 23 -alles aus einem Polyolefinmaterial spritzgeformt -ist das durchgehende Rohr R mittels Abschlussstück 31 im Verschlussblock 32 axial gelagert.

Das Rohr R besteht aus rostfreiem Spezialstahl und ist hinten schräg geschnitten. Es ist fix im Abschlussstück 31 befestigt, d.h. bei der üblichen Verwendung des Gerätes bewegt sich das genannte Abschlussstück gegenüber dem Rohr nicht. Das Rohrende ist hinten etwa bündig mit dem Ende des Spritzenkörpers.

Das Abschlussstück 31 ist so ausgebildet, dass Flüssigkeit, speziell Blut, von innerhalb des genannten Stückes immer zum Innenraum des durchgehenden Rohres gelangen kann. Dies ist deutlich aus den Figuren 4a und 4b ersichtlich.

Das Abschlussstück 31 ist axial verschiebbar - aber flüssigkeitsdicht -im Verschlussblock 32 gelagert. Bei der in Figur 2 dargestellten Anordnung ist das Abschlussstück nach hinten gezogen angeordnet, d.h. die flüssigkeitsführende Verbindung von Kanülenhalter -Innenraum und innerem Hohlraum 33 des Verschlussblockes 32 zum Innenraum des Abschlussstückes 31 ist unterbrochen. Das Abschlussstück 31 besteht z.B. aus spritzgeformtem Polyacrylat und ist auf das Rohr R aufgespannt bzw. thermisch aufgezogen.

Der Verschlussblock 32 besteht aus dem gleichen oder aus ähnlichem Werkstoff wie der Spritzenkörper und wird auch analog hergestellt. Er ist vorn im Spritzenkörper fixiert, z.B. durch Einspannen oder Kleben, und dichtet an den Innenwänden des Spritzenkörpers ab. Flüssigkeit, speziell Blut, kann nur auf dem in Figur 2 mittels eines Pfeiles angezeigten Weg zum Abschlussstück 31 und somit in das durchgehende Rohr gelangen.

Als zweites wird nun ein Auffangbehälter AB behändigt und gegebenenfalls ausgepackt. Derselbe liegt grundsätzlich gemäss Figur 3 vor, d.h. die Abdichtung 12 ist vorn am Hohlkolben 11 fixiert und überragt denselben. Der Abdichtungs-Kolben 13 ist ebenfalls vorn, an die Abdichtung anliegend, angeordnet. In dieser praktisch unveränderlichen Anordnung werden die Auffangbehälter angeliefert.

Der Auffangbehälter wird nun so wie er vorliegt und gegebenenfalls nach Entfernung einer Verpackung von hinten her in den Spritzenkörper eingedrückt. Das schräg geschnittene Rohrende wird dabei durch die speziell dafür ausgebildete Vertiefung 121 zur Schwachstelle 122 in der Abdichtung 12 geführt und durchstösst diese bei Einwirkung von genügend Druck. Nach dem Durchtreten des Rohrendes durch die Abdichtung 12 stösst dasselbe bei weiterer Druckeinwirkung auf den Auffangbehälter den Abdichtungskolben 13 im Hohlzylinder 11 nach hinten und erzeugt so im grösserwerdenen Volumen zwischen Abdichtung 12 und Kolben 13 einen Unterdruck. Durch der dabei in Richtung Gerätespitze auf das durchgehende Rohr ausgeübten Druck (aufgrund der Haftungskräfte zwischen elastischer Abdichtung und Rohr) wird das Rohr mit dem Abschlussstück 31 nach vorn gedrückt und es öffnet sich somit die flüssigkeitsführende Verbindung von Kanüleninnenraum über Hohlraum 33 des Verschlussblockes 31 -Leitungen im Abschlussstück - durchgehendes Rohr in den Raum im Auffangbehälter AB zwischen Abdichtung 12 und Kolben 13. Dieser füllt sich somit bis zum gewünschten Niveau, was einfach durch den auf den Auffangbehälter ausgeübten Druck erreicht wird. So kann auch die Geschwindigkeit der Blutentnahme äusserst einfach reguliert werden.

Der Hohlzylinder 11 besteht vorteilhafterweise ebenfalls aus spritzgeformten Polyolefinen, wie auch der Kolben 13. Der letztere ist so ausgeformt, dass er durch Einwirken der bei der üblichen Anwendung des erfindungsgemässen Gerätes erreichten Druckdifferenz relativ leicht in Richtung der Längsachse bewegt wird und dabei aber zwischen sich und der Innenwand des Hohlzylinders 11 hinsichtlich Flüssigkeiten abdichtet -z.B. mittels der in Figur 3 angedeuteten zwei Dichtungsringe.

Der Zustand des erfindungsgemässen Gerätes am Ende der Blutentnahme wird grundsätzlich in Figur 1 festgehalten: Der Auffangbehälter AB ist bis zum Anschlag der Andichtung in Form des Dichtungspfropfens 12 an den Verschlussblock 31 in den Spritzenkörper SK eingedrückt worden. Dabei ist die maximal mögliche Blutmenge (nicht eingezeichnet) entnommen worden.

Der mittels des durchgehenden Rohres zu durchstossende, fixe Abdichtungspfropfen 12 besteht aus einem natürlichen oder synthetischen Kautschuk; wesentlich ist, dass dieses Material - wie alle andern im Gerät verwendeten Werkstoffe - in den üblichen Vorrichtungen keimfrei gemacht werden kann.

Der verwendete Auffangbehälter kann gegebenenfalls die üblichen Mittel zur Konservierung und/oder Umsetzung des entnommenen Blutes enthalten.

Sobald die gewünschte Blutmenge vorliegt, wird -drittens -der Auffangbehälter AB wieder nach hinten aus dem Spritzenkörper herausgezogen.

Dabei geschieht zweierlei: Durch Zugwirkung auf das Rohr in Richtung hinteres Ende des Gerätes werden das durchgehende Rohr wie auch der Abschlussteil 31 nach hinten bewegt und die flüssigkeitsführende Verbindung zwischen einerseits Kanüleninnenraum und bzw. innerem Hohlraum 33 des Verschlussblockes 32 und andererseits Innenleitungen des Abschlussteils 31 und Rohr unterbrochen. Die Möglichkeit, Blut durch die Kanüle wieder auszustossen besteht nicht.

Beim weiteren Zurückziehen des Auffangbehälters wird schliesslich auch das durchgehende Rohr aus dem Pfropfen 12 gezogen, der, da aus elastischem Material bestehend, anschliessend dicht abschliesst.

Die Abschnitte 2 und 3 der geschilderten Blutentnahme können ersichtlicherweise beliebig oft bei Benutzung der gleichen eingestochenen Kanüle und Spritzenkörper plus durchgehendem Rohr ausgeführt werden.

Nach Abschluss der Blutentnahme wird -bei Benutzung eines Sets -der gefüllte Auffangbehälter jeweils in sein Abteil in der Verpackung zurückgelegt, die Kanüle fachgerecht wieder herausgezogen und zusammen mit dem Spritzenkörper mit durchgehendem Rohr in das abtrennbare Abteil der Verpackung abgelegt, das letztgenannte Abteil vom Rest des Sets abgetrennt (beispielsweise durch Abreissen), der Rest des Sets beschrieben und gegebenenfalls versandt.

## Ansprüche

1. Gesichertes Blutentnahmegerät, umfassend als Hauptbestandteile einen Spritzenkörper (SK) mit Kanülenhalter, einen in den Spritzenkörper einzudrückenden Auffangbehälter (AB) sowie ein durchgehendes Rohr (R) dadurch gekennzeichnet, dass der Auffangbehälter in Form eines Hohlzylinders (11) mit vorn angebrachter, fixierter Abdichtung (12) aus elastischem Material, mit innen angebrachtem, gegenüber der Kolbeninnenwand beweglichem Abdichtungskolben (13) und mit hinten vorgesehener, Oeffnung (14), welche beim Eindrücken des Hohlzylinders in den Spritzenkörper unter Verschiebung des Abdichtungskolbens (13) das Austreten der Luft aus dem Auffangbehälter ermöglicht, vorliegt, dass der Spritzenkörper an seinem hinteren Ende Grifflappen (21), die bei der Verwendung des Gerätes z.B. durch Zeig-und Mittelfinger gehalten werden und an der Stirnfläche (22) einen Kanülenhalter (23) aufweist und dass er am hinteren Ende offen ausge bildet ist und dass das durchgehende Rohr mittels eines Abschlussstückes (31) in einem Verschlussblock (32) in Richtung Geräte-Längsachse beweglich gelagert ist, wobei der Verschlussblock (32) dicht an der vorderen Abschliessfläche (22) befestigt ist und eine flüssigkeitsführende Verbindung zwischen Kanüleninnenraum und innerem Hohlraum (33) des Verschlussblockes (32) schafft und wobei das am durchgehenden Rohr dicht fixierte Abschlussstück (31) bei Druck auf das Rohr in Richtung Gerätespitze eine flüssigkeitsführende Verbindung zwischen dem Rohr-Innenraum und dem inneren Hohlraum (33) schafft, und bei Zug auf das durchgehende Rohr diese Verbindung flüssigkeitsdicht abschliesst.

2. Blutentnahmegerät gemäss Patentanspruch 1, dadurch gekennzeichnet, dass der Auffangbehälter aus einem Hohlzylinder (11) aus spritzgeformtem Polyolefin-Material besteht und eine Abdichtung (12) in Form eines Pfropfens aus keimfrei zu machendem Synthese-oder Naturkautschuk aufweist, welche Abdichtung eine der Lage des durchgehenden Rohres entsprechende Vertiefung (121) und Schwachstelle (122) zur Führung des genannten Rohres und zur Bestimmung der Durchstichstelle aufweist, wobei die Abdichtung über das vordere Ende des Auffangbehälters hinausragt und zugleich zur nichtdichtenden Führung dieses Behälters im Spritzenkörper dient und dass der Auffangbehälter einen inneren beweglichen, ebenfalls aus Polyolefinen spritzgeformten Abdichtungskolben (13) hat, der in seinem Aussendurchmesser und in seiner Anlagefläche derart an die Innenwand des Hohlzylinders (11) angepasst ist, dass er bei den auftretenden Druckunterschieden für die üblicherweise aufzunehmenden Flüssigkeiten dicht ist; der Auffangebehälter ist zudem auf die hintere Abschlussfläche mit Oeffnung (14) abstellbar.

3. Blutentnahmegerät gemäss Patentanspruch 1 oder 2, dadurch gekennzeichnet, dass der Spritzenkörper aus Polyolefin-Material druckgeformt ist und einen material-und formmässig integrierten Kanülenhalter (23) zur Aufnahme einer Metall-Kanüle oder einer Kanüle aus Metallspitze und elastischer Zuleitung zum Kanülenhalter aufweist.

4. Blutentnahmegerät gemäss Patentanspruch 1 oder gemäss den Patentansprüchen 2 und 3, dadurch gekennzeichnet, dass das durchgehende Rohr aus Spezialstahl besteht, dass es vorn ein Abschlussstück (31), gegebenenfalls aus spritzgeformten Acryl-Kunststoffen, aufweist, welcher Abschluss die flüssigkeitsführende Verbindung von Rohr -Innenraum zum inneren Hohlraum (33) des Verschlussblockes herstellt bzw. unterbricht und dicht und nichtbeweglich am Rohrende aufgezogen ist, dass es mittels des Abschlussstückes (31) im Verschlussblock (32) axial beweglich gelagert ist, wobei der Verschlussblock vorn, z.B. innen an der vorderen Abschliessfläche (22) des Spritzenkörpers befestigt ist, die flüssigkeitsführende Verbindung zwischen dem Kanüleninnenraum und -bei Druck auf das durchgehende Rohr -seinem inneren Hohlraum (33) schafft und wobei das genannte Abschlussstück (31) flüssigkeitsdicht im genannten Hohlraum (33) in Achsrichtung beweglich ist und dass das durchgehende Rohr hinten schräg angeschnitten ist, um so bei der Verwendung des Gerätes das Einstechen des genannten Rohres in die Dünnstelle (122) der Abdichtung zu erleichtern.

5. Blutentnahmegerät gemäss einem der Patentansprüche 1 oder 4, dadurch gekennzeichnet, dass es in Form eines Sets vorliegt, in welchem Set der Spritzenkörper mit darin befestigtem durchgehendem Rohr und daneben der bzw. die Auffangbehälter verpackt ist (sind).

6. Blutentnahmegerät gemäss Patentanspruch 5, dadurch gekennzeichnet, dass der Spritzenkörper mit durchgehendem Rohr in einem separaten Abteil gelagert ist, welches vom übrigen Set-Behälter leicht abtrennbar ist, dass der oder die Auffangbehälter gemeinsam in einem zweiten Abteil gelagert ist (sind), welches zweite Abteil zudem -nach Abtrennen des separaten Abteils mit dem (gebrauchten) Spritzenkörper -beschriftbar und versendbar ist.

7. Verwendung des gesicherten Blutentnahmegerätes gemäss einem der vorangehenden Patentansprüche zur Blutentnahme bei Menschen.

8. Verwendung gemäss Patentanspruch 7, gekennzeichnet durch
-Einführen der fixiert oder beweglich am Blutentnahmegerät angebrachten Kanüle in die Vene,
-Eindrücken eines Auffangbehälters in den Spritzenkörper, wobei das durchgehende Rohr die vordere Abdichtung (12) aus elastischem Material durchsticht und den inneren, beweglichen Abdichtungskolben (13) im Hohlzylinder des Auffangbehälters nach hinten schiebt und so darin einen Unterdruck erzeugt, welcher Unterdruck, da mit dem Druck auf das Rohr in Richtung Gerätespitze das Abschlussstück (31) nach vorn geschoben wird und so die Verbindung Kanüle -Innenraum (33) - Rohr geschaffen wird, das Aufsaugen von Blut in den Auffangbehälter bewirkt wird und
-Zurückziehen des Auffangbehälters bei Erreichen der gewünschten Blutmenge im Auffangbehälter, wodurch sowohl Blutzufuhr wie auch -austritt gestoppt werden und wodurch, beim vollständigen Zurückziehen des Auffangbehälters, ein hermetisch verschlossener sowie auf die hintere Abschlussfläche stellbarer Behälter mit dem entnommenen Blut erhalten wird.

9. Verwendung gemäss Patentanspruch 8, dadurch gekennzeichnet, dass die eigentliche Blutentnahme bei gleicher eingestochener Kanüle mit gleichem Spritzenkörper und durchgehendem Rohr nacheinander unter Verwendung von mehreren Auffangbehältern, beispielsweise unter Verwendung des Sets gemäss Patentanspruch 6, ausgeführt wird.

# Fig. 1

# Fig. 2

0 224 604

Fig. 3

Fig. 4a

Fig. 4b

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,Y | EP-A-0 150 127 (BILLBATE LTD.) * Zusammenfassung; Seite 2, Zeilen 8-11; Seite 3, Zeile 4 - Seite 4, Zeile 2; Seite 5, Zeilen 13-24; Abbildung 2 * | 1,4,7 | A 61 B 5/14 |
| A | | 2 | |
| | --- | | |
| Y | US-A-3 159 159 (M.J. COHEN) * Spalte 2, Zeilen 29-54; Spalte 3, Zeilen 30-50; Spalte 4, Zeilen 16-61; Spalte 6, Zeilen 17-22; Abbildungen 1-3 * | 1,4,7-9 | |
| | --- | | |
| A | US-A-4 020 831 (S.L. ADLER) * Zusammenfassung; Spalte 3, Zeilen 27-40,63-68; Spalte 4, Zeilen 16-36,50-62; Spalte 5, Zeilen 13-32; Abbildungen 1-7 * | 1-3,5, 7-9 | RECHERCHIERTE SACHGEBIETE (Int. Cl.4) A 61 B |
| | --- | | |
| A | US-A-3 942 514 (R.W. OGLE) * Spalte 2, Zeilen 15-36; Spalte 3, Zeilen 7-17,39-56; Abbildungen 1-5 * | 1,2,7 | |
| | --- | | |
| A | FR-A-1 416 487 (P. CINQUALBRE) * Insgesamt * | 5-7,9 | |
| | --- -/- | | |

Der vorliegende Recherchenbericht wurde für alle Patentansprüche erstellt.

| Recherchenort DEN HAAG | Abschlußdatum der Recherche 30-07-1986 | Prüfer RIEB K.D. |
|---|---|---|

## EINSCHLÄGIGE DOKUMENTE

Seite 2

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | FR-A-1 361 093 (BECTON DICKINSON & CO.) <br> * Seite 1, rechte Spalte; Zeilen 4-22; Seite 2, linke Spalte, Zeilen 23-31, rechte Spalte; Zeilen 27,28; Seite 3, rechte Spalte, Zeilen 23-34; Abbildungen 1-6 * | 3,5-7, 9 | |

-----

**RECHERCHIERTE SACHGEBIETE (Int. Cl.4)**

Der vorliegende Recherchenbericht wurde für alle Patentansprüche erstellt.

| Recherchenort | Abschlußdatum der Recherche | Prüfer |
|---|---|---|
| DEN HAAG | 30-07-1986 | RIEB K.D. |